# EUROPEAN PATENT APPLICATION

(11) **EP 3 266 368 A1**
(43) Date of publication of application: **10.01.2018**
(21) Application number: 16177784.2
(22) Date of filing: 04.07.2016
(51) Int. Cl.: A61B 5/00, A61B 5/087, A61B 5/08, A61B 5/097, A61B 5/085

(54) **RHINOMANOMETRY DEVICE**

(71) Applicant: Rhinolab GmbH, 79108 Freiburg (DE)
(72) Inventor: Bäumle, Marc-Aurel, 79106 Freiburg (DE); Bank, Philipp, 79112 Freiburg (DE)
(74) Representative: Maucher Jenkins Patent- und Rechtsanwälte

(57) **Abstract**

The present invention relates to a rhinomanometry device comprising: a measurement device for measuring at least one set of values of one or more parameters relating to a respiratory function of the nose of a patient; a memory for storing measurement values; a processing and control device for processing an output of the measurement device, the processing and control device to: determine if the set of measured values satisfies at least a first predetermined criterion; control the memory to store the set of measured values, and control the measurement device to stop the measurement in response to determining that the set of measured values satisfies at least a second predetermined criterion.

## Description

### Technical Field

The present invention relates to a rhinomanometry device.

### Background

Rhinomanometry is a form of manometry used to evaluate the respiratory function of the nose of a patient. Rhinomanometry measures pressure and volume flow during normal inspiration (inhalation) and expiration (exhalation) through the nose. For example, increased pressure during respiration is usually an indicator of increased resistance to airflow through nasal passages (nasal blockage).

Rhinomanometry may be used to measure only one nostril at a time or both nostrils simultaneously.

In order to obtain a reliable result, it is essential to evaluate a plurality of consecutive inhalation and exhalation cycles. That is, a practitioner applies a rhinomanometer to the nose of the patient and measures the pressure and volume flow during a plurality of consecutive inhalation and exhalation cycles. In conventional systems, the practitioner manually starts and terminates the measurement. The measurements are then processed by a processing device connected to the rhinomanometer. If the measurements satisfy certain criteria indicating normal inhalation and exhalation cycles, the measured values are stored and displayed for diagnosis by the practitioner. Otherwise, the measurement values are rejected and the practitioner needs to repeat the measurement.

Another problem associated with conventional rhinomanometry systems is that the measurement values can vary depending on "healthy" characteristics of the nose such as its size. In other words, two different noses both having a normal respiratory function deliver different measurement values. This can make it difficult to distinguish "good" measurements from measurements representing unnatural inhalations or exhalations, for example due to nervousness of the patient.

The present invention aims to address these problems and to provide a rhinomanometry device that enables an improved collection of measurement values. In particular, the present invention aims to provide a rhinomanometry device that is capable of determining when measurement values are available that enable a reliable and objective diagnosis of the respiratory function of the nose of a patient.

### Summary

According to one aspect of the present invention, there is provided a rhinomanometry device comprising: a measurement device for measuring at least one set of values of one or more parameters relating to a respiratory function of the nose of a patient; a memory for storing measurement values; a processing and control device for processing an output of the measurement device, the processing and control device to: determine if the set of measured values satisfies at least a first predetermined criterion; control the memory to store the set of measured values, and control the measurement device to stop the measurement in response to determining that the set of measured values satisfies at least a second predetermined criterion.

Accordingly, the present invention enables the collection of measurement values that have a predefined minimum quality. Once sufficient measurement values having that quality are available, the measurement is automatically stopped. The user, e.g. practitioner, no longer has to manually repeat the measurement if the quality of the measurement is found to be insufficient during visual inspection of the displayed results. Also, the patient is no longer required to undergo repeated measurement cycles until the measurements are found to be satisfactory, which repetition could lead to weariness or nervousness of the patient and deterioration of the results. In addition, the present invention provides for a reduction in the amount of measurement data that needs to be stored and processed.

Preferably, each set of measured values corresponds to a measuring period defined by an inhalation phase and an exhalation phase. Accordingly, measurement values of sufficient quality representing at least one breath, consisting of an inhalation phase and an exhalation phase, are obtained.

In an embodiment, the parameters include volume flow and/or pressure of air in the nose of the patient. Measurement of these parameters enables the determination of a Nasal Valve Index (NVI), as described below.

In an embodiment, said first criterion includes one or more thresholds for the volume flow and/or the pressure of air in the nose of the patient and/or the duration of the measuring period. In particular, the processing device is to determine a maximum of the volume flow of air in the nose of the patient during the inhalation phase, wherein said first criterion is satisfied if the maximum of the volume flow exceeds a predetermined first threshold, and/or the processing device to determine a minimum of the volume flow of air in the nose of the patient during the exhalation phase, wherein said first criterion is satisfied if the minimum of the volume flow falls below a predetermined second threshold. Additionally or alternatively, said first criterion is satisfied if the duration of the measurement period exceeds a predetermined third threshold. Thereby, it is made sure that only measurement values corresponding to sufficiently strong and long breaths are taken into account for subsequent storage and processing.

In an embodiment, the first threshold is at least 30 ccm/s, preferably 50 ccm/s, and can reach a threshold of 600 ccm/s (50% of a maximum scale value) and/or wherein the second threshold is less than -30 ccm/s, preferably -50 ccm/s, and can reach a threshold of -600 ccm/s (50% of a minimum scale value) and/or wherein the third threshold is at least 0.5, 0.8, 1.0, or 1.2s and can reach a threshold of 5s. These thresholds have been found to deliver useful measurement results.

In another embodiment, the processing device is to determine the deviation of one or more or all of a newly measured set of values with one or more values from a previously measured set of values, wherein the second criterion is satisfied if the deviation is below a predetermined fourth threshold. In particular, the processing device is to determine the deviation of one or more or all of a newly measured set of values with one or more values from each of at least the two or three last-measured sets of values, wherein the second criterion is satisfied if the deviation in respect of each of the at least two or three last-measured sets of values is below the fourth threshold, wherein the fourth threshold is less than 50%, preferably no more than 30% and ideally no more than 10%. For example, in an embodiment, the second criterion is satisfied if the maxima and/or minima of the volume flow and/or the duration of the combined inhalation and exhalation phases included in or represented by the respective stored sets of measured values do not deviate from one another by more than the fourth threshold. In this embodiment, measurements representing a sequence of relatively similar breaths can be obtained.

The rhinomanometry device may further comprise an input device for entering and/or adjusting the parameters defining said first and second criteria. Accordingly, it is possible to adjust the quality of measurements required to trigger an automatic stop.

The rhinomanometry device further comprises an input device for enabling a user to manually stop the measurement, wherein, in response to a manual stop, the processing and control device is to control the memory to store the last set of measured values prior to the manual stop that represents a full measuring period. Preferably, in response to a manual stop, the processing and control device is to determine if at least the two last sets of measured values prior to the manual stop satisfy the second criterion, and to control the memory to store the at least two last sets of measured values in response to determining that the second criterion is satisfied. Accordingly, in response to a manual stop, the rhinomanometry device determines if the measurement results obtained prior to the manual stop have a minimum quality. If this is the case, rather than discarding the measurement values, they are stored and processed further.

In an embodiment, the parameters include the volume flow and pressure of air in the nose of the patient, wherein the measurement device is to measure a plurality of said parameter values over a period of time corresponding to an inhalation phase, and wherein the processing device is to determine: a first maximum value corresponding to the maximum of measured volume flow values; a second maximum value corresponding to the maximum of measured pressure values; a first value corresponding to the area of a rectangle defined by said first and second maximum values, a zero volume flow value and a zero pressure value; and a second value corresponding to an integration of the area under a curve defined by consecutive values of the volume flow over the pressure between the zero pressure value and the maximum pressure value; a third value corresponding to the integration of the area under a curve defined by consecutive values of the volume flow over the pressure between the maximum pressure value and the zero pressure value; a fourth value corresponding to the difference between the second and third values; an fifth value corresponding to the difference between the first and fourth values; and an output value corresponding to the ratio of the first and fifth values; and an output device for generating an output signal indicative of the output value.

In this embodiment, a Nasal Valve Index (NVI) is determined. It has been found that the NVI is an objective indicator of the state of the respiratory function of a patient's nose. In particular, the NVI is represents a ratio rather than an absolute value and is therefore independent of certain factors that could influence the result, e.g. the size of the nose of the patient.

The present invention also provides for a determination of the NVI independently of an automatic measurement stop. Accordingly, in another aspect of the present invention, there is provided a rhinomanometry device comprising: a measurement device for measuring the values of one or more parameters relating to a respiratory function of the nose of a patient, wherein the parameters include the volume flow and pressure of air in the nose of the patient, and wherein the measurement device is to measure a plurality of said parameter values over a period of time corresponding to an inhalation phase; a processing device for processing an output of the measurement device to determine: a first maximum value corresponding to the maximum of measured volume flow values; a second maximum value corresponding to the maximum of measured pressure values; a first value corresponding to the area of a rectangle defined by said first and second maximum values, a zero volume flow value and a zero pressure value; and a second value corresponding to an integration of the area under a curve formed by consecutive values of the volume flow over the pressure between the zero pressure value and the maximum pressure value; a third value corresponding to the integration of the area under a curve formed by consecutive values of the volume flow over the pressure between the maximum pressure value and the zero pressure value; a fourth value corresponding to the difference between the second and third values; a fifth value corresponding to the difference between the first and fourth values; and an output value corresponding to the ratio of the first and fifth values; and an output device for generating an output signal indicative of the output value.

### Brief Description of the Drawings

The present invention may be better understood from reading the following description of non-limiting embodiments, with reference to the attached drawings, wherein:
Fig. 1 schematically shows a rhinomanometry device in accordance with an embodiment of the present invention;
Figs. 2a and 2b illustrate diagrams of volume flow and pressure values from exemplary measurements using the rhinomanometry device of Fig. 1; and
Figs. 3a and 3b illustrate diagrams of the volume flow over the pressure of an exemplary averaged measurement using the rhinomanometry device of Fig. 1.

### Detailed Description of Exemplary Embodiments

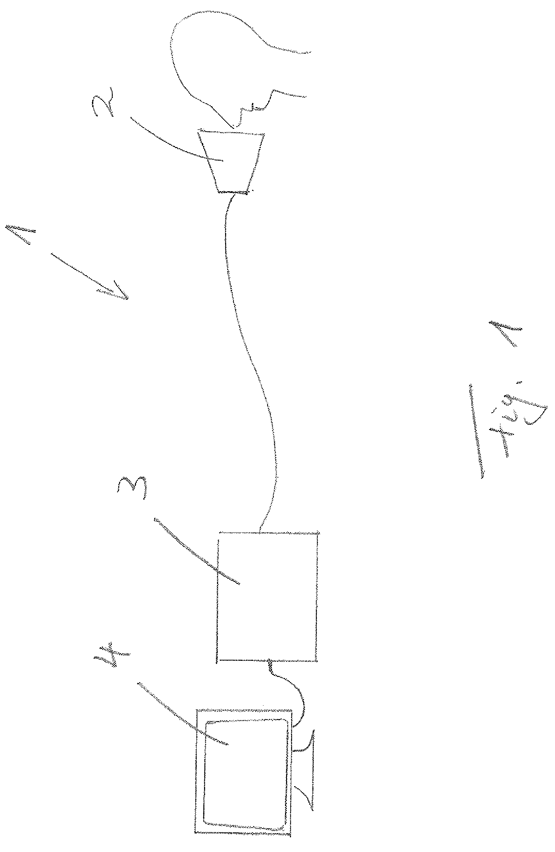

Fig. 1 schematically shows a rhinomanometry device 1 in accordance with an embodiment of the present invention. The rhinomanometry device 1 comprises a rhinomanometer 2 to measure the volume flow and the pressure of air in the nose of a patient. The rhinomanometry device 1 also comprises a computer 3 that is connected to the rhinomanometer 2. The computer 3 that is adapted to process and store measurement values from rhinomanometer 2. The computer 3 comprises a display 4 for displaying the measurement values to a user.

Rhinomanometers are known as such and therefore not describe herein.

In operation, the rhinomanometry device 1 measures and records a sequence of values of the volume flow and the pressure of air in the nose of a patient. For example, the volume flow and pressure of air can be measured every 5 ms. The measurement frequency is adjustable.

Each breath consists of an inhalation and an exhalation and includes a plurality of measurement values of the volume flow and the pressure. At the end of each breath, the volume flow of the air, coming from a negative value, becomes zero, i.e. crosses the x-axis of a diagram representing the volume flow of air over time.

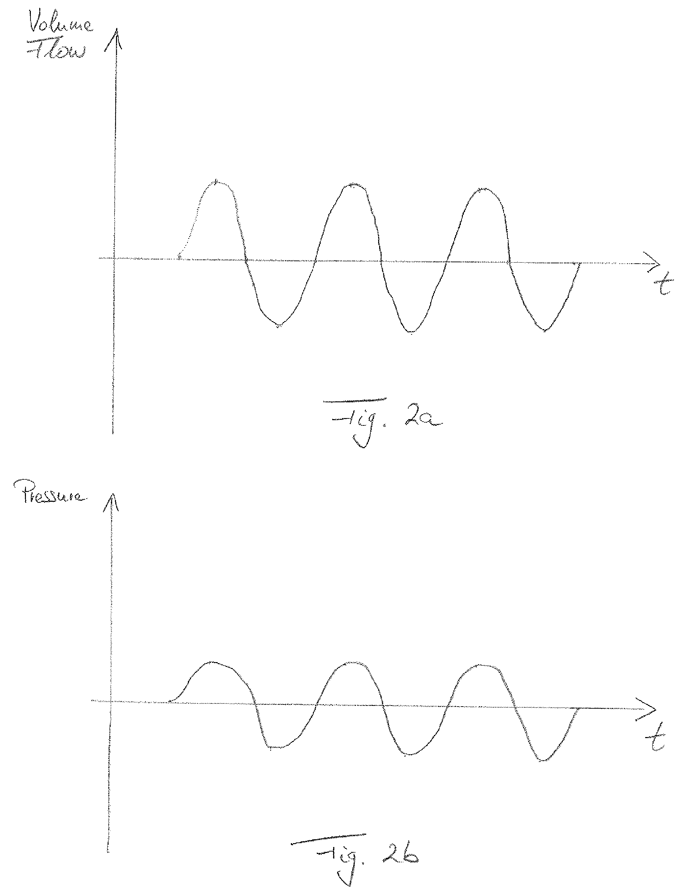

Figs. 2a und 2b illustrate diagrams of volume flow and pressure values, respectively, from exemplary measurements. Fig. 2a illustrates the volume flow of air over four breaths. Fig. 2ab illustrates the pressure of air over the same period.

For each breath, the maxima and minima of the volume flow and pressure are determined. In addition, the duration of each breath is determined. Measurements that satisfy predetermined criteria are added to a list of records representing "good" breaths, i.e. breaths that enable a practitioner to diagnose the condition of the patient's respiratory system. In an exemplary embodiment, these criteria are satisfied if the duration of the breath is at least 1s, the maximum volume flow during inhalation is at least 50 ccm/s and the minimum volume flow during exhalation is -50 ccm/s or less.

Measurements of a subsequent breath are added to the list if the above criteria are met and, additionally, there is a sufficient similarity with the measurements of one or more previous breaths. In an exemplary embodiment, there is sufficient similarity if the duration as well as the maximum and minimum volume flows do not vary by more than 30%.

If a predetermined number of consecutive breaths satisfy the above criteria, the measurement is stopped. For example, if three consecutive breaths are sufficiently similar to one another, then the corresponding measurements are "approved" and the measurement is terminated. The measurement values of the approved measurements can then be displayed for further use, e.g. diagnosis by a practitioner.

In an embodiment of the invention, the measurement values are processed to determine a Nasal Valve Index (NVI). In a first step, the measurement values of the approved measurements are averaged, thereby to determine an average set of values corresponding to one breath. This set of values is divided in two sub-sets corresponding to the inhalation and the exhalation phase of the breath, respectively. This is done on the basis of the zero-point of the volume flow. For determining the NVI, only the measurement values of the inhalation phase are used. The inhalation phase is further sub-divided into two portions, a first portion corresponding to the phase before the maximum pressure value within the inhalation phase is attained, and a second portion corresponding to the phase after the maximum pressure value within the inhalation phase is attained.

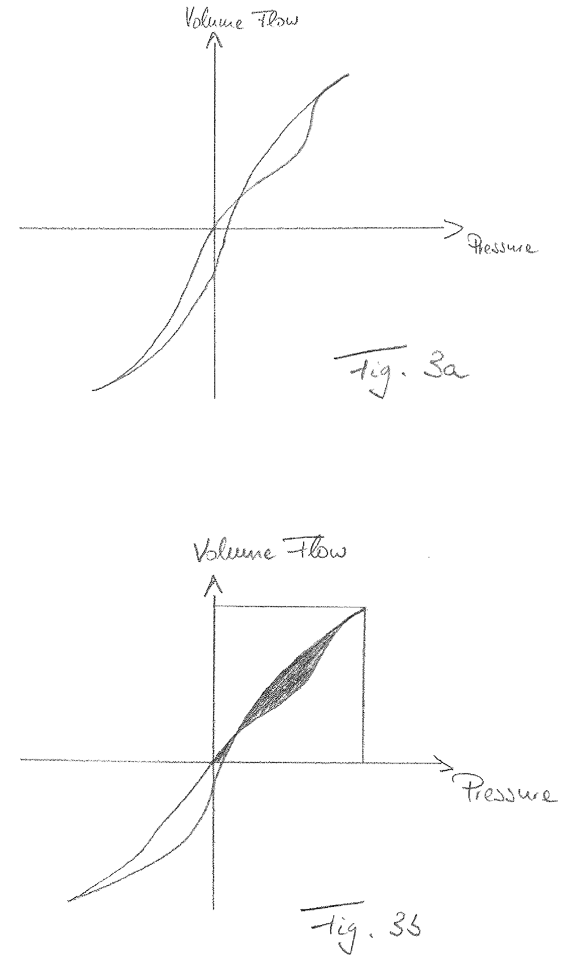

Figs. 3a and 3b illustrate diagrams of the volume flow over the pressure of an exemplary measurement averaged as described above. The curves in the first quadrant of the diagram represent the measurement values of the inhalation phase.

In order to determine the NVI, the areas underneath the curves corresponding to the above-mentioned first and second portions of the inhalation phase are determined and subtracted from one another. The result corresponds to the highlighted area between the curves shown in Fig. 3b.

The ratio of this area to the total area corresponding to the rectangle in Fig. 3b, i.e. the maximum pressure multiplied with the maximum volume flow at the maximum pressure, when represented as a percentage, results in the NVI.

The NVI is independent of time and absolute volume flow and pressure values. Instead, the NVI indicates the deviation of the curves from one another before and after the maximum pressure value. It has been found that the NVI constitutes a useful and reliable representation of the state of a patient's respiratory system.

The description of embodiments has been presented for purposes of illustration and description. Suitable modifications and variations to the embodiments may be performed in light of the above without departing from the scope of protection as determined by the claims.

## Claims

1. A rhinomanometry device comprising:
a measurement device for measuring at least one set of values of one or more parameters relating to a respiratory function of the nose of a patient;
a memory for storing measurement values;
a processing and control device for processing an output of the measurement device, the processing and control device to:
determine if the set of measured values satisfies at least a first predetermined criterion;
control the memory to store the set of measured values, and
control the measurement device to stop the measurement in response to determining that the set of measured values satisfies at least a second predetermined criterion.

2. The rhinomanometry device of claim 1, wherein each set of measured values corresponds to a measuring period defined by an inhalation phase and an exhalation phase.

3. The rhinomanometry device of claim 1 or 2, wherein said parameters include volume flow and/or pressure of air in the nose of the patient.

4. The rhinomanometry device of any preceding claim, wherein said first criterion includes one or more thresholds for the volume flow and/or the pressure of air in the nose of the patient and/or the duration of the measuring period.

5. The rhinomanometry device of any preceding claim, the processing device to determine a maximum of the volume flow of air in the nose of the patient during the inhalation phase, wherein said first criterion is satisfied if the maximum of the volume flow exceeds a predetermined first threshold, and/or the processing device to determine a minimum of the volume flow of air in the nose of the patient during the exhalation phase, wherein said first criterion is satisfied if the minimum of the volume flow falls below a predetermined second threshold.

6. The rhinomanometry device of any preceding claim, wherein said first criterion is satisfied if the duration of the measurement period exceeds a predetermined third threshold.

7. The rhinomanometry device of any preceding claim, wherein the first threshold is at least 30 ccm/s, preferably 50 ccm/s, and can reach a threshold of up to 600 ccm/s or 50% of a maximum scale value, and/or wherein the second threshold is less than -30 ccm/s, preferably -50 ccm/s, and can reach a threshold of down to -600 ccm/s or 50% of a minimum scale value, and/or wherein the third threshold is at least 0.5 or 0.8 or 1.0 or 1.2s and can reach a threshold of up to 5s.

8. The rhinomanometry device of any preceding claim, wherein the processing device is to determine the deviation of one or more or all of a newly measured set of values with one or more values from a previously measured set of values, wherein the second criterion is satisfied if the deviation is below a predetermined fourth threshold.

9. The rhinomanometry device of any preceding claim, wherein the processing device is to determine the deviation of one or more or all of a newly measured set of values with one or more values from each of at least the two or three last-measured sets of values, wherein the second criterion is satisfied if the deviation in respect of each of the at least two or three last-measured sets of values is below the fourth threshold, wherein the fourth threshold is less than 50%, and preferably no more than 30% or 10%.

10. The rhinomanometry device of any preceding claim, wherein the second criterion is satisfied if the maxima and/or minima of the volume flow and/or the duration of the combined inhalation and exhalation phases included in or represented by the respective stored sets of measured values do not deviate from one another by more than the fourth threshold.

11. The rhinomanometry device of any preceding, further comprising an input device for entering and/or adjusting the parameters defining said first and second criteria.

12. The rhinomanometry device of any preceding claim, further comprising an input device for enabling a user to manually stop the measurement, wherein, in response to a manual stop, the processing and control device is to control the memory to store the last set of measured values prior to the manual stop that represents a full measuring period.

13. The rhinomanometry device of any preceding claim, wherein, in response to a manual stop, the processing and control device is to determine if at least the two last sets of measured values prior to the manual stop satisfy the second criterion, and to control the memory to store the at least two last sets of measured values in response to determining that the second criterion is satisfied.

14. The rhinomanometry device of any preceding claim, wherein the parameters include the volume flow and pressure of air in the nose of the patient, and wherein the measurement device is to measure a plurality of said parameter values over a period of time corresponding to an inhalation phase, and wherein the processing device is to determine:
a first maximum value corresponding to the maximum of measured volume flow values;
a second maximum value corresponding to the maximum of measured pressure values;
a first value corresponding to the area of a rectangle defined by said first and second maximum values, a zero volume flow value and a zero pressure value; and
a second value corresponding to an integration of the area under a curve defined by consecutive values of the volume flow over the pressure between the zero pressure value and the maximum pressure value;
a third value corresponding to the integration of the area under a curve defined by consecutive values of the volume flow over the pressure between the maximum pressure value and the zero pressure value;
a fourth value corresponding to the difference between the second and third values;
a fifth value corresponding to the difference between the first and fourth values; and
an output value corresponding to the ratio of the first and fifth values; and
an output device for generating an output signal indicative of the output value.

15. A rhinomanometry device comprising:
a measurement device for measuring the values of one or more parameters relating to a respiratory function of the nose of a patient, wherein the parameters include the volume flow and pressure of air in the nose of the patient, and wherein the measurement device is to measure a plurality of said parameter values over a period of time corresponding to an inhalation phase;
a processing device for processing an output of the measurement device to determine:
a first maximum value corresponding to the maximum of measured volume flow values;
a second maximum value corresponding to the maximum of measured pressure values;
a first value corresponding to the area of a rectangle defined by said first and second maximum values, a zero volume flow value and a zero pressure value; and
a second value corresponding to an integration of the area under a curve formed by consecutive values of the volume flow over the pressure between the zero pressure value and the maximum pressure value;
a third value corresponding to the integration of the area under a curve formed by consecutive values of the volume flow over the pressure between the maximum pressure value and the zero pressure value;
a fourth value corresponding to the difference between the second and third values;
a fifth value corresponding to the difference between the first and fourth values; and
an output value corresponding to the ratio of the first and fifth values; and
an output device for generating an output signal indicative of the output value.
